# EUROPÄISCHE PATENTANMELDUNG

(11) **EP 3 561 814 A1**
(43) Veröffentlichungstag der Anmeldung: **30.10.2019**
(21) Anmeldenummer: 18169430.8
(22) Anmeldetag: 26.04.2018
(51) Int. Cl.: G16H 10/20

(54) **STEIGERUNG DER ADHÄRENZ IN KLINISCHEN STUDIEN**

(71) Anmelder: Bayer AG, 51373 Leverkusen (DE)
(72) Erfinder: LAUBE, Kathrin, 10318 Berlin (DE); SPANNAGEL, Flora Isabelle, 40545 Düsseldorf (DE); BRUSNIKIN, Denis, 10016 New York (US); CHARTIER, Taylor, 94109 San Francisco (US); LIM, Jin Cheng, Centre East (SG)
(74) Vertreter: BIP Patents

(57) **Zusammenfassung**

Die vorliegende Erfindung betrifft eine Vorrichtung, ein Verfahren und ein Computerprogrammprodukt zur Unterstützung von Personen, die an einer klinischen Studie teilnehmen.

## Beschreibung

Die vorliegende Erfindung betrifft eine Vorrichtung, ein Verfahren und ein Computerprogrammprodukt zur Unterstützung von Personen, die an einer klinischen Studie teilnehmen.

Bei der Durchführung von klinischen Studien ist es wichtig, dass die Versuchspersonen die einzunehmenden Arzneimittelportionen in der Menge, zu den Zeiten und unter den Bedingungen des jeweiligen Studienprotokolls einnehmen (Adhärenz).

Jede Abweichung von dem Studienprotokoll kann die Qualität der erhobenen Daten negativ beeinträchtigen.

Die Aufgabe der vorliegenden Erfindung besteht darin, Personen, die an einer klinischen Studie als Studienteilnehmer teilnehmen, bei der Adhärenz zu unterstützen.

Gelöst wird diese Aufgabe durch die Gegenstände der unabhängigen Patentansprüche. Bevorzugte Ausführungsformen finden sich in den abhängigen Patentansprüchen.

Ein erster Gegenstand der vorliegenden Erfindung ist eine Vorrichtung umfassend
- einen Signalgeber
- eine Bildaufnahmeeinheit
- eine Verarbeitungseinheit
- einen Datenspeicher
- eine Zeitbestimmungseinheit
- eine Eingabeeinheit
- eine Ausgabeeinheit
wobei in dem Datenspeicher Zeitpunkte und/oder Zeitspannen gespeichert sind, zu denen ein Nutzer eine oder mehrere Arzneimittelportion im Rahmen einer klinischen Studie einnehmen soll
wobei die Verarbeitungseinheit konfiguriert ist, aus dem Datenspeicher die Zeitpunkte und/oder Zeitspannen auszulesen
wobei die Verarbeitungseinheit konfiguriert ist, eine aktuelle Zeit über die Zeitbestimmungseinheit zu erfassen
wobei die Verarbeitungseinheit konfiguriert ist, die aus dem Datenspeicher ausgelesenen Zeitpunkte und/oder Zeitspannen mit der aktuellen Zeit zu vergleichen
wobei die Verarbeitungseinheit konfiguriert ist, den Signalgeber zu veranlassen, bei Eintreten eines definierten Ereignisses ein Signal abzugeben
wobei die Verarbeitungseinheit konfiguriert ist, die Ausgabeeinheit zu veranlassen, bei Eintreten eines definierten Ereignisses eine Mitteilung über eine erforderliche Einnahme einer oder mehrerer Arzneimittelportion auf auszugeben
wobei die Verarbeitungseinheit konfiguriert ist, die Ausgabeeinheit zu veranlassen, eine Abbildung von der einen oder den mehreren Arzneimittelportionen auf einem Bildschirm anzuzeigen.

Über den Signalgeber kann die erfindungsgemäße Vorrichtung ein Signal an einen Nutzer der Vorrichtung abgegeben. Bei diesem Signal kann es sich um ein akustisches Signal (z.B. einen Ton, eine Tonfolge und/oder eine Sprachnachricht), um ein taktiles Signal (z.B. ein Vibrieren) und/oder ein optisches Signal (z.B. ein Aufblinken einer Lichtquelle) oder dergleichen handeln. Der Signalgeber soll die Aufmerksamkeit des Nutzers auf die Vorrichtung lenken. Ein oder mehrere Signale werden vorzugsweise dann erzeugt, wenn der Nutzer eine Aktion durchführen soll, zu der er weitere Informationen z.B. auf einem Bildschirm der Vorrichtung angezeigt bekommt. Die genannten Signalgeber sind heute standardmäßig in mobilen und stationären Computern verbaut.

Bei der Bildaufnahmeeinheit handelt es sich um eine Einheit zur Erzeugung von digitalen Bildaufnahmen. Solche Bildaufnahmeeinheiten sind heute standardmäßig in mobilen und stationären Computern als Kameras verbaut.

Bei der Verarbeitungseinheit handelt es sich um einen oder mehrere Mikrocontroller, die Daten entgegennehmen, verarbeiten und ausgeben können. Verarbeitungseinheiten sind das Herzstück heutiger Computer.

Bei dem Datenspeicher kann es sich um einen oder mehrere Einheiten zur dauerhaften Speicherung digitaler Daten handeln. Dauerhaft bedeutet, dass die gespeicherten Daten zu späteren Zeitpunkten immer wieder ausgelesen werden können. Ferner ist der Datenspeicher so ausgelegt, dass neue Daten hinzugefügt werden können. Solche Datenspeicher sind heute standardmäßig in mobilen und stationären Computern als Kameras verbaut.

Die Zeitbestimmungseinheit dient der Bestimmung der aktuellen Zeit. Es kann sich dabei um eine Uhr mit Datumsfunktion handeln, die Bestandteil der erfindungsgemäßen Vorrichtung ist. Denkbar ist aber auch, dass die Vorrichtung die aktuelle Zeit über eine Empfangseinheit empfangen kann.

Über die Eingabeeinheit werden Informationen und/oder Daten in die erfindungsgemäße Vorrichtung eingebracht. Üblicherweise dient die Eingabeeinheit (zusammen mit der Ausgabeeinheit) auch als Kommunikationsschnittstelle mit dem Nutzer der erfindungsgemäßen Vorrichtung. Die Eingabeeinheit kann eine Tastatur, eine Maus, einen Touchscreen, ein Mikrofon, eine Netzwerkverbindung, eine Verbindung zu einem Datenspeicher, ein Verbindung zu einem Gerät und/oder dergleichen umfassen.

Über die Ausgabeeinheit werden Informationen und/oder Daten ausgegeben. Die Ausgabeeinheit kann einen Bildschirm, einen Drucker, einen Lautsprecher, einen Datenspeicher, eine Verbindung zu einem Netzwerk, eine Verbindung zu einem Gerät und/oder dergleichen umfassen.

Vorzugsweise umfasst die erfindungsgemäße Vorrichtung ferner eine Empfangseinheit. Mit der Empfangseinheit kann die Vorrichtung Informationen und/oder Daten von anderen entfernten Computersystemen und/oder Geräten empfangen. Ebenso verfügt die erfindungsgemäße Vorrichtung vorzugsweise auch über eine Sendeeinheit, mit der Informationen und/oder Daten an einen entfernten Computer und/oder ein anderes Gerät übermittelt werden können. Der Empfang und/oder das Senden erfolgen vorzugsweise über eine Funkverbindung wie beispielsweise über ein Mobilfunknetz, eine WLAN-Verbindung, über Bluetooth oder dergleichen. Derartige Empfangseinheiten sind heute standardmäßig in mobilen und stationären Computern verbaut.

Ein weiterer Gegenstand der vorliegenden Erfindung ist ein Computerprogrammprodukt umfassend einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:
- Ermitteln von Zeitpunkten und/oder Zeitspannen, zu denen ein Nutzer eine oder mehrere Arzneimittelportion im Rahmen einer klinischen Studie einnehmen soll
- Ausgeben einer Mitteilung über eine erforderliche Einnahme der oder der mehreren Arzneimittelportionen bei Eintreten eines definierten Ereignisses
- Anzeigen einer Abbildung der einen oder der mehreren Arzneimittelportionen auf einem Bildschirm.

Das Computerprogramm wird üblicherweise in einen Arbeitsspeicher der erfindungsgemäßen Vorrichtung geladen. Dort veranlasst es die erfindungsgemäße Vorrichtung, einen oder mehrere Schritte des erfindungsgemäßen Verfahrens auszuführen.

Ein weiterer Gegenstand der vorliegenden Erfindung ist ein Verfahren umfassend die Schritte:
- Ermitteln von Zeitpunkten und/oder Zeitspannen, zu denen ein Nutzer eine oder mehrere Arzneimittelportion im Rahmen einer klinischen Studie einnehmen soll
- Ausgeben einer Mitteilung über eine erforderliche Einnahme der oder der mehreren Arzneimittelportionen bei Eintreten eines definierten Ereignisses
- Anzeigen einer Abbildung der einen oder der mehreren Arzneimittelportionen auf einem Bildschirm.

Die Erfindung wird nachstehend näher erläutert, ohne zwischen den Erfindungsgegenständen (Verfahren, Vorrichtung, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt) sie erfolgen.

Die Studienteilnehmer werden während der klinischen Studie durch ein Computerprogramm unterstützt. Das Computerprogramm kann als On-Premise-Lösung auf einem Computersystem des Studienteilnehmers installiert sein. Denkbar ist auch, dass das Computerprogramm als "Software-as-a-Service" auf einem Computersystem des Anbieters installiert ist, und der Nutzer über eine Schnittstelle wie beispielsweise ein Web-Interface auf das Computerprogramm zugreift.

Es ist denkbar, dass das Computerprogramm auf einem stationären und/oder mobilen Computersystem installiert ist oder von einem stationären und/oder mobilen Computersystem auf das Computerprogramm zugegriffen wird.

Vorzugsweise nutzt der Studienteilnehmer ein mobiles Computersystem wie beispielsweise ein Smartphone oder einen Tablet-Computer oder dergleichen, um das Computersystem zu nutzen.

Das Computersystem, das der Studienteilnehmer nutzt, um auf das Computerprogramm zuzugreifen, wird in dieser Beschreibung auch als Vorrichtung bezeichnet.

Für die Begleitung während der klinischen Studie stellt das Computerprogramm (bzw. die erfindungsgemäße Vorrichtung) eine Reihe von Funktionalitäten bereits, die nachfolgend anhand von Figuren näher beschrieben werden. Die in den Figuren gezeigten Merkmale stellen lediglich Beispiele für bevorzugte Ausführungsformen dar; sie sind nicht so zu verstehen, dass die Erfindung auf diese Merkmale oder Merkmalskombinationen beschränkt werden soll.

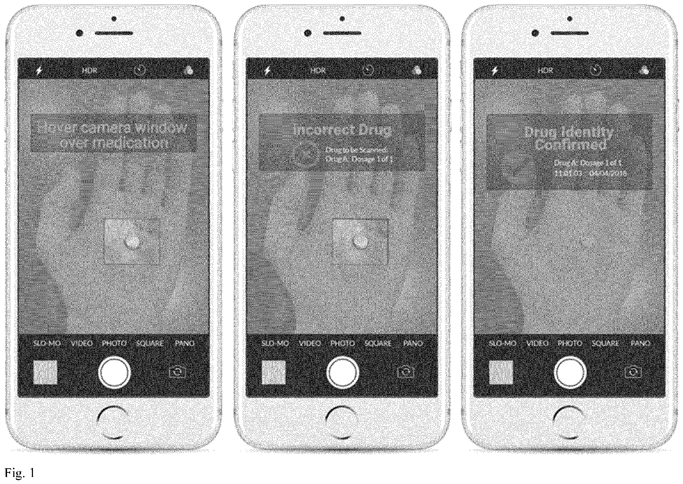

Figur 1 zeigt beispielhaft die Funktion zur Erkennung einer Arzneimittelportion. Der Nutzer verwendet die Kamera seines Smartphone und präsentiert die Arzneimittelportion. Die auf dem Bildsensor der Kamera abgebildete Abbildung wird auf dem Bildschirm des Smartphones dargestellt, so dass der Nutzer sieht, was auf dem Kamerachip abgebildet wird. Das Smartphone ist so konfiguriert, dass es eine Arzneimittelportion in der Abbildung erkennen kann. Mittels eines Rahmens wird dem Nutzer angezeigt, dass das Vorhandensein einer Arzneimittelportion in der Abbildung erkannt worden ist. Vorzugsweise werden Bereiche außerhalb des Rahmens mit weniger Kontrast oder in Graustufen angezeigt, um die Aufmerksamkeit des Nutzers auf die Arzneimittelportion zu lenken. Ziel ist es, eine möglichst hochaufgelöste und nicht verwackelte Bildaufnahme der Arzneimittelportion zu erzielen. Es ist denkbar, dass sich die Farbe des Rahmens an die Qualität der Bildaufnahme anpasst. Ist zum Beispiel die Arzneimittelportion in der Abbildung zu klein dargestellt oder zu dunkel abgebildet oder zu hell abgebildet oder werden zu starke Bewegungen, die zu verwackelten Aufnahmen führen könnten, vollzogen, kann der Rahmen in einer gelben oder einer anderen Farbe dargestellt werden. Ist die Arzneimittelportion jedoch in einer definierten Mindestgröße in der Abbildung zu erkennen und sind die Relativbewegungen zwischen Arzneimittelportion und Kameraoptik vernachlässigbar und entspricht die Belichtung definierten Werten, kann der Rahmen in einer blauen oder einer anderen Farbe dargestellt werden. Die Farbe des Rahmens oder eine andere vorzugsweise visuell dargestellte Information gibt dem Nutzer somit die Anweisung, die Arzneimittelportion anders zu präsentieren, um die Qualität der Bildaufnahme zu steigern, bis eine Qualität erreicht ist, die mit einer definierten Wahrscheinlichkeit dazu führt, dass die Arzneimittelportion erkannt wird.

Es ist denkbar, dass der Nutzer einen virtuellen Knopf auf dem Bildschirm des Smartphones oder einen realen Knopf am Smartphone drücken muss, um eine digitale Bildaufnahme zu erzeugen. Vorzugsweise ist das Smartphone so konfiguriert, dass es laufend digitale Bildaufnahmen erzeugt, im Arbeitsspeicher auf die Anwesenheit einer Arzneimittelportion analysiert, und, falls das Vorhandensein einer Arzneimittelportion erkannt worden ist, eine digitale Bildaufnahme erzeugt, die dann einer weiteren Analyse in Bezug auf die Identifikation der Arzneimittelportion unterzogen wird. Die automatische Erzeugung einer digitalen Arzneimittelportion hat den Vorteil, dass der Nutzer lediglich die Kamera vor die Arzneimittelportion halten muss und gegebenenfalls die Position der Arzneimittelportion gegenüber der Kamera variieren muss, bis eine digitale Bildaufnahme mit einer für die nachfolgende Identifizierung ausreichenden Qualität erzeugt wird. Da bereits eine Hand die Arzneimittelportion und die andere Hand das Smartphone hält, könnte es für den Nutzer eine Schwierigkeit bedeuten, in dieser Situation auch noch einen (virtuellen oder realen) Knopf drücken zu müssen. Das Drücken eines Knopfes könnte auch zu einer Verwackelung führen.

Die erzeugte digitale Arzneimittelportion kann über eine Netzwerkverbindung (z.B. WLAN oder das Mobilfunknetz) an einen Server übermittelt werden, der dann die Identifikation der Arzneimittelportion in der Bildaufnahme übernimmt. Nach der Identifikation kann der externe Server über das Netzwerk eine Mitteilung an das Smartphone senden, in der Informationen darüber enthalten sind, welche Arzneimittelportion identifiziert worden ist. Vorzugsweise erfolgt die Identifikation der abgebildeten Arzneimittelportion auf dem Smartphone (der erfindungsgemäßen Vorrichtung).

Zur Identifikation können bekannte Verfahren der Bildanalyse (Mustererkennung, selbstlernende Systeme wie Deep Neural Networks und dergleichen) eingesetzt werden.

Die identifizierte Arzneimittelportion wird mit der einzunehmenden Arzneimittelportion verglichen. Stimmt die identifizierte Arzneimittelportion mit der einzunehmenden Arzneimittelportion überein, wird dem Nutzer über den Bildschirm des Smartphones angezeigt, dass er die richtige Arzneimittelportion vorliegen hat und diese nun einnehmen kann. Stimmt die identifizierte Arzneimittelportion nicht mit der einzunehmenden Arzneimittelportion überein, wird dem Nutzer über den Bildschirm des Smartphones angezeigt, dass er nicht die richtige Arzneimittelportion vorliegen hat und diese nicht einnehmen sollte. Vorzugsweise werden ihm über den Bildschirm Bilder der einzunehmenden Arzneimittelportion angezeigt, so dass er weiß, wie die einzunehmende Arzneimittelportion aussieht. Er kann dann eine andere Arzneimittelportion nehmen und diese erneut und wie beschrieben dem Smartphone präsentieren und damit zur Prüfung vorlegen.

Die Mitteilung über die richtige oder falsche Arzneimittelportion kann durch optische Darstellungen unterstützt werden.

Ist die richtige Arzneimittelportion präsentiert worden, so kann der Zeitpunkt der analysierten Bildaufnahme als Zeitpunkt für die Einnahme der Arzneimittelportion festgehalten (gespeichert) werden (Zeitstempel). Neben dem Zeitstempel wird festgehalten, welche Arzneimittelportion vorgelegen hat. Denkbar ist aber auch, dass der Nutzer die tatsächliche Einnahme gegenüber dem Smartphone durch Drücken eine virtuellen oder realen Knopfes zu bestätigen hat; in diesem Fall wird vorzugsweise der Zeitpunkt des Drückens des Knopfes als Zeitstempel festgehalten.

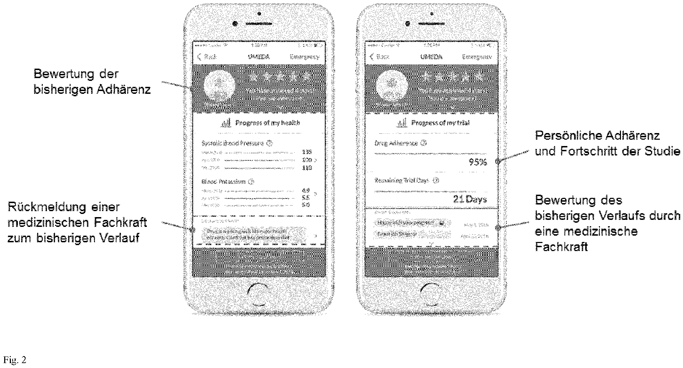

Figur 2 zeigt verschiedene Anzeigen auf dem Smartphone des Nutzers, die dem Nutzer gegenüber angezeigt werden können, um seine Adhärenz zu steigern oder eine hohe Adhärenz zu fördern.

Auf der linken Seite ist ein Beispiel einer Anzeige gezeigt. Im oberen Bereich der Anzeige ist eine Zusammenfassung des bisherigen Studienerfolgs in Form einer Bewertung mit 0 bis 5 Sternen gezeigt. Je mehr Sterne hervorgehoben sind, desto erfolgreicher verläuft die Studie. Als Erfolg der Studie kann der Grad der Adhärenz (z.B. das Verhältnis von in der Vergangenheit eingenommenen zu einzunehmenden Arzneimittelportionen) und/oder der gesundheitliche Verlauf des Studienteilnehmers herangezogen werden.

Im mittleren Bereich der Anzeige werden Gesundheitsparameter des Studienteilnehmers angezeigt (im vorliegenden Beispiel der systolische Blutdruck und der Kaliumgehalt des Blutes). Die Anzeige wird grafisch unterstützt, indem Werte, die in einem Bereich liegen, der als "gesund" erachtet wird, grün dargestellt werden, während Werte, die in einem Bereich liegen, der als "krank" erachtet wird, rot dargestellt werden, und Werte, die sich im Übergangsbereich zwischen "grün" und "rot" befinden, beispielsweise "gelb" oder "orange" dargestellt werden. Es können auch zeitliche Verläufe dargestellt werden, damit der Studienteilnehmer erkennen kann, wie sich Werte im Verlauf der Studie verändert haben.

Im unteren Bereich der Anzeige können Rückmeldungen von medizinischen Fachkräften zum bisherigen Studienverlauf und -fortschritt angezeigt werden.

Auf der rechten Seite der Figur 2 ist eine alternative Anzeige gezeigt. Der Grad der Adhärenz wird hier durch einen Balken zusammen mit einer Prozentzahl dargestellt. Eine hundertprozentige Adhärenz bedeutet, dass der Studienteilnehmer alle Aktionen, die das Studienprotokoll für ihn vorschreiben, durchgeführt hat (z.B. Einnahme von Arzneimittelportionen, Ausfüllen von Patientenfragebögen, Durchführen von Messungen, Aufsuchen von Ärtzen, Aufsuchen von Laboren zur Ermittlung weiterer gesundheitlicher Parameter, Bewegung oder andere unterstützende Maßnahmen, Einhalten von Ernährungsvorschriften oder -tipps usw.). Zusätzlich wird dem Studienteilnehmer angezeigt, wie viele Tage der Studie bereits vergangen und/oder noch zu bewältigen sind.

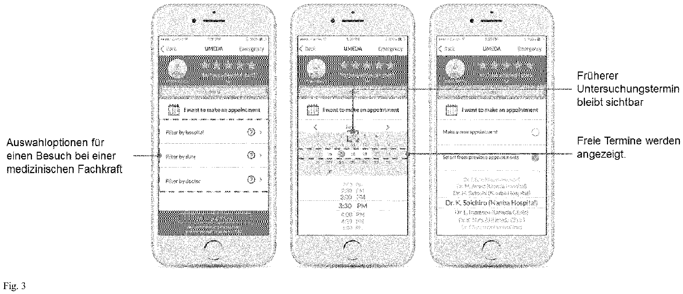

Figur 3 zeigt beispielshaft, wie ein Studienteilnehmer mittels des Computerprogramms/der Vorrichtung einen Termin mit einer medizinischen Fachkraft abstimmen kann.

Üblicherweise muss ein Studienteilnehmer ab und zu eine medizinische Fachkraft aufsuchen, um sich medizinisch untersuchen zu lassen. Eine solche Untersuchung kann zum Ziel haben, die Wirkungen und/oder Nebenwirklungen von verabreichten Arzneimitteln zu überprüfen.

Es ist aber auch denkbar, dass der Studienteilnehmer von sich aus einen Termin mit einer medizinischen Fachkraft vereinbaren möchte, um beispielsweise über Effekte der Studie und/oder Erfahrungen mit der Studie zu sprechen oder Rat einzuholen.

Die Terminvereinbarungsfunktion kann für beide Szenarien (und weitere denkbare Szenarien) genutzt werden.

In einer bevorzugten Ausführungsform kann der Studienteilnehmer den Zeitpunkt einer Untersuchung zumindest in einem gewissen Ausmaß frei wählen und/oder den Ort für die Untersuchung zumindest in einem gewissen Ausmaß frei wählen und/oder die medizinische Fachkraft, die die Untersuchung durchführt, zumindest in einem gewissen Ausmaß frei wählen.

In Figur 3 ist in der Anzeige auf der linken Seite zu erkennen, dass der Studienteilnehmer, verfügbare Krankenhäuser (Ort der Untersuchung), Zeitpunkte (Datum) und medizinische Fachkräfte (Arzt) anzeigen lassen kann.

Dem Studienteilnehmer kann ein Kalender angezeigt werden (mittlere Anzeige), in der die Untersuchungstermine der Vergangenheit eingetragen sind und freie zukünftige Termin z.B. grün markiert sind. Auf der rechten Seite wird eine Liste von medizinischen Fachkräften angezeigt, bei denen in der Vergangenheit schon einmal ein Untersuchungstermin stattgefunden hat.

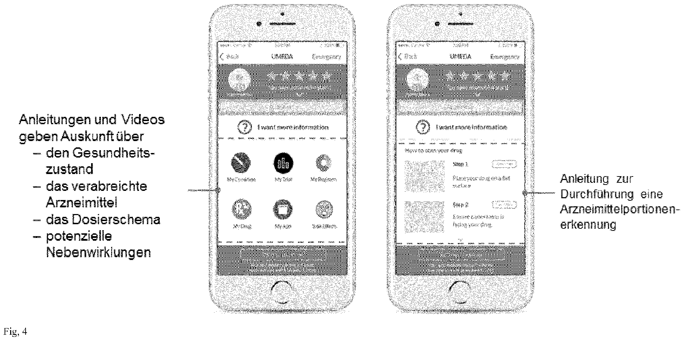

Figur 4 zeigt beispielhaft zwei Anzeigen des Smartphones zu Informationen, die ein Studienteilnehmer abrufen kann. Er kann Informationen über die Studie selbst, über Arzneimittel, die im Rahmen der Studie verabreicht werden, über potenzielle Nebenwirkungen, über seinen Gesundheitszustand und/oder über das bei ihm verwendete Dosierschema anzeigen lassen. Zudem kann er sich Informationen zur Bedienung des Computerprogramms, wie beispielsweise zur Durchführung einer Arzneimittelportionenerkennung anzeigen lassen (rechte Anzeige).

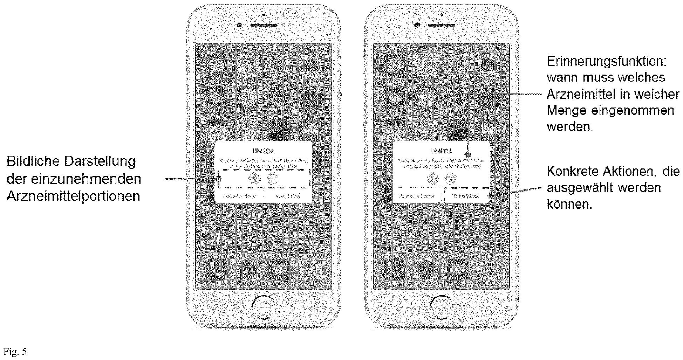

Figur 5 zeigt beispielhaft zwei Anzeigen, bei denen dem Studienteilnehmer Mitteilungen übersandt werden. In der rechten Anzeige ist eine Erinnerungsmitteilung angezeigt. Sie soll den Studienteilnehmer an die Einnahme einer oder mehrerer Arzneimittelportionen erinnern. Vorzugsweise wird konkret beschrieben, wann er welche Arzneimittelportionen unter welchen Bedingungen (z.B. nüchtern oder zum Essen oder mit Flüssigkeit oder dergleichen) einnehmen sollte. Vorzugsweise werden dem Studienteilnehmer reale Bildaufnahmen oder am Computer erzeugte Bilder der Arzneimittelportionen angezeigt, die er einnehmen soll, damit er direkt erkennen kann, welche und wie viele Arzneimittelportionen er einnehmen soll. Er kann dann eine konkrete Aktion auswählen, zum Beispiel, bestätigen, dass er die Arzneimittelportion unmittelbar nehmen wird, oder zu einem späteren Zeitpunkt noch einmal erinnert werden möchte. Bei der Wahl einer späteren Erinnerung zeigt ihm das Computerprogramm vorzugsweise an, bis wann er die Arzneimittelportionen noch nehmen kann, ohne dass es einen negativen Einfluss auf die Adhärenz und/oder das Studienprotokoll und/oder die Datenqualität und/oder die gewünschte Wirkung und/oder eine unerwünschte Nebenwirkung hat. In der linken Anzeige ist zu erkennen, dass der Studienteilnehmer eine Erinnerung erhält, wenn er die Einnahme einer Arzneimittelportion nicht zu einem definierten Zeitpunkt gegenüber der Vorrichtung / dem Computerprogramm bestätigt hat. Er kann dann entweder bestätigen, dass er die Arzneimittelportion eingenommen hat (aber die Einnahme nicht bestätigt hat) oder er kann sich anzeigen lassen, was er zu tun hat.

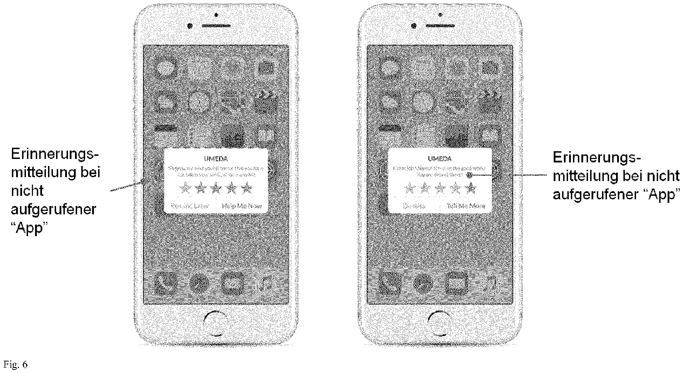

Vorzugsweise ist die erfindungsgemäße Vorrichtung so konfiguriert, dass sie dem Studienteilnehmer auch dann Mitteilungen anzeigt, wenn er die entsprechende Applikation ("App") auf dem Smartphone gar nicht aufgerufen hat. Dies ist beispielhaft in Fig. 6 illustriert. Auf der linken Seite ist gezeigt, dass der Studienteilnehmer eine Mitteilung erhält, nachdem er die App für eine definierte Zeit nicht aufgerufen hat. Es wird ihm Hilfe angeboten, für den Fall, dass er Schwierigkeiten beim Starten der App hat. Auf der rechten Seite erhält der Studienteilnehmer Rückmeldung zu seiner vorbildlichen Adhärenz.

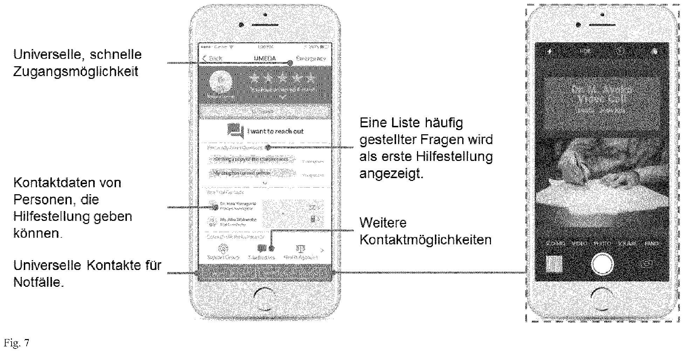

Figur 7 zeigt beispielhaft eine Notfallfunktion, die seitens des Computerprogramms zur Verfügung gestellt wird. Es ist denkbar, dass im Rahmen der Studie eine Situation auftritt, in der der Studienteilnehmer Bedarf an einem kurzfristigen Austausch mit einer medizinischen Fachkraft hat; z.B., weil er sich ungut fühlt oder Nebenwirkungen aufgetreten sind oder er einfach dringende Fragen hat.

Für diesen Zweck wird vorzugsweise in jeder Anzeige des Computerprogramms eine Notruffunktion als virtueller Knopf (*button*) bereitgestellt, den der Studienteilnehmer z.B. über den Touchscreen seines Smartphones betätigen kann. Es wird eine Liste häufig gestellter Fragen (FAQ-Liste) als eine erste Hilfestellung angezeigt. Falls diese Informationen dem Studienteilnehmer keine ausreichende Hilfestellung geben, kann der Studienteilnehmer aus einer Liste von Personen eine Person auswählen, die er kontaktieren möchte. Vorzugsweise handelt es sich dabei um medizinische Fachkräfte. Der Studienteilnehmer kann auswählen, ob er die jeweilige Person über E-Mail, Telefon, SMS, einen Messenger-Dienst kontaktieren möchte. Vorzugsweise wird dem Studienteilnehmer angezeigt, wo sich die jeweilige Person gerade befindet (z.B. damit er sie persönlich besuchen kann) und/oder ob die Person gerade verfügbar ist. Ein Telefongespräch mit der Person wird vorzugsweise mit einer Videofunktion unterstützt, so dass der Studienteilnehmer ggf. Symptome der Person zeigen kann (z.B. einen Hautausschlag oder dergleichen). Der Studienteilnehmer kann den jeweiligen Notfall auch weiter eingrenzen, z.B. durch Drücken virtueller Knöpfe für einen akuten Notfall oder eine Nebenwirkung, die gemeldet werden soll/kann. Vorzugsweise wird er durch Drücken eines der genannten virtuellen Knöpfe direkt mit einer Person verbunden, die entsprechende Hilfestellung geben kann. Weitere Kontaktmöglichkeiten können angeboten werden.

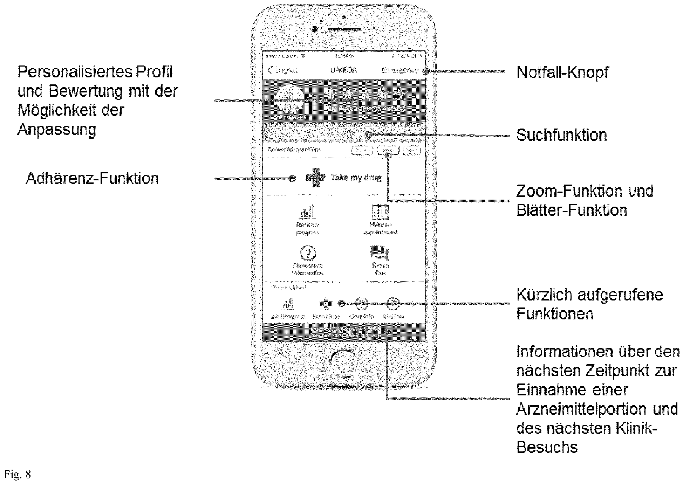

Figur 8 zeigt eine weitere Anzeige des erfindungsgemäßen Computerprogramms, in der verschiedene wichtige Funktionen dargestellt und aufrufbar sind. Im unteren Bereich der Anzeige befindet sich ein Feld mit Informationen, z.B. wann die nächste Arzneimittelportion einzunehmen ist und/oder wann der nächste Besuch bei einer medizinischen Fachkraft (z.B. ein Besuch in einer Klinik) ansteht.

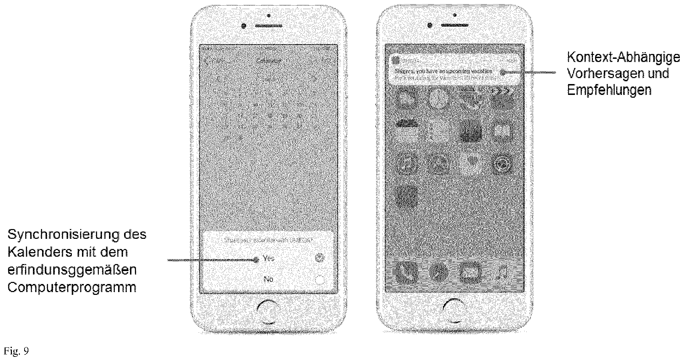

Figur 9 zeigt eine Synchronisationsfunktion. Jedes Smartphone verfügt heutzutage über eine App mit einer Kalenderfunktion. Der Nutzer trägt in den Kalender üblicherweise Termine ein. Das erfindungsgemäße Computerprogramm bietet die Möglichkeit einer Synchronisierung von Funktionalitäten mit dem Kalender. Zunächst einmal können anstehende Termine im Zusammenhang mit der klinischen Studie (z.B. Besuch bei einer medizinischen Fachkraft) oder Termine zur Einnahme einer Arzneimittelportion oder dergleichen in den Kalender eingetragen werden; der Studienteilnehmer muss also nicht zwei verschiedene Kalender führen. Daneben liest das erfindungsgemäße Computerprogramm den Kalender aus und interpretiert die Einträge. Zum Beispiel erkennt das erfindungsgemäße Computerprogramm anstehende Reisen anhand von typischerweise mit Reisen in Verbindung stehenden Begriffen. Erkennt das erfindungsgemäße Computerprogramm, dass eine Reise unmittelbar bevorsteht, erzeugt es eine Mitteilung, die dem Versuchsteilnehmer angezeigt wird. Zum Beispiel kann die Mitteilung den Studienteilnehmer daran erinnern, genügend Arzneimittelportionen einzupacken, damit der Studienteilnehmer auch auf Reisen genügend Arzneimittelportionen zu Verfügung hat.

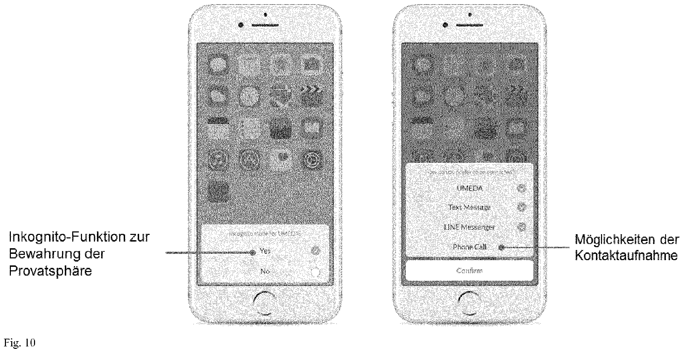

Figur 10 zeigt eine Inkognito-Funktion. Es ist denkbar, dass der Studienteilnehmer nicht möchte, dass andere Personen, die ggf. Zugriff auf das Smartphone haben, erfahren, dass der Studienteilnehmer an einer Studie teilnimmt. Mit der Inkognito-Funktion wird das erfindungsgemäße Computerprogramm in einen Ruhemodus versetzt: es versendet keine Mitteilungen an den Studienteilnehmer, die von anderen bemerkt werden könnten. Grundsätzlich bietet das Computerprogramm die Möglichkeit, die Arten der Kontaktaufnahmen zu wählen, z.B. aus der Liste: Mitteilung über das Computerprogramm (UMEDA), Textnachrichten, Messenger-Dienst, Telefonanruf.

## Patentansprüche

1. Vorrichtung umfassend
• einen Signalgeber
• eine Bildaufnahmeeinheit
• eine Verarbeitungseinheit
• einen Datenspeicher
• eine Zeitbestimmungseinheit
• eine Eingabeeinheit
• eine Ausgabeeinheit
wobei in dem Datenspeicher Zeitpunkte und/oder Zeitspannen gespeichert sind, zu denen ein Nutzer eine oder mehrere Arzneimittelportion im Rahmen einer klinischen Studie einnehmen soll
wobei die Verarbeitungseinheit konfiguriert ist, aus dem Datenspeicher die Zeitpunkte und/oder Zeitspannen auszulesen
wobei die Verarbeitungseinheit konfiguriert ist, eine aktuelle Zeit über die Zeitbestimmungseinheit zu erfassen
wobei die Verarbeitungseinheit konfiguriert ist, die aus dem Datenspeicher ausgelesenen Zeitpunkte und/oder Zeitspannen mit der aktuellen Zeit zu vergleichen
wobei die Verarbeitungseinheit konfiguriert ist, den Signalgeber zu veranlassen, bei Eintreten eines definierten Ereignisses ein Signal abzugeben
wobei die Verarbeitungseinheit konfiguriert ist, die Ausgabeeinheit zu veranlassen, bei Eintreten eines definierten Ereignisses eine Mitteilung über eine erforderliche Einnahme einer oder mehrerer Arzneimittelportion auf auszugeben
wobei die Verarbeitungseinheit konfiguriert ist, die Ausgabeeinheit zu veranlassen, eine Abbildung von der einen oder den mehreren Arzneimittelportionen auf einem Bildschirm anzuzeigen.

2. Vorrichtung gemäß Anspruch 1,
wobei die Verarbeitungseinheit konfiguriert ist, die Ausgabeeinheit zu veranlassen, einen Nutzer dazu aufzufordern, eine definierte Arzneimittelportion zu präsentieren,
wobei die Verarbeitungseinheit konfiguriert ist, die Bildaufnahmeeinheit zu veranlassen, Bildaufnahmen zu erzeugen,
wobei die Verarbeitungseinheit konfiguriert ist, die erzeugten Bildaufnahmen zu analysieren und die Anwesenheit einer Arzneimittelportion zu erkennen,
wobei die Verarbeitungseinheit konfiguriert ist, eine Bildaufnahme mit einer als anwesend erkannten Arzneimittelportion zu analysieren und die Arzneimittelportion zu identifizieren,
wobei die Verarbeitungseinheit konfiguriert ist, die identifizierte Arzneimittelportion mit der definierten Arzneimittelportion zu vergleichen,
wobei die Verarbeitungseinheit konfiguriert ist, die Ausgabeeinheit zu veranlassen, dem Nutzer eine Mitteilung über das Ergebnis des Vergleichens zu übermitteln.

3. Vorrichtung gemäß Anspruch 2,
wobei die Verarbeitungseinheit konfiguriert ist, die Ausgabeeinheit zu veranlassen, die erzeugten Bildaufnahme als Livebilder auf einem Bildschirm anzuzeigen
wobei die Verarbeitungseinheit konfiguriert ist, die Ausgabeeinheit zu veranlassen, in den Livebildern anzuzeigen, ob eine Anwesenheit einer Arzneimittelportion erkannt oder nicht erkannt wird.

4. Vorrichtung gemäß einem der Ansprüche 2 oder 3,
wobei die Verarbeitungseinheit konfiguriert ist, für den Fall, dass die identifizierte Arzneimittelportion mit der definierten Arzneimittelportion übereinstimmt,
- die Ausgabeeinheit zu veranlassen, dem Nutzer eine Mitteilung zu übermitteln, wobei die Mitteilung eine Aufforderung zur Einnahme der Arzneimittelportion umfasst,
- die aktuelle Zeit über die Zeitbestimmungseinheit zu erfassen,
- eine Information über die Einnahme der Arzneimittelportion zu dem aktuellen Zeitpunkt in dem Datenspeicher zu speichern.

5. Vorrichtung gemäß einem der Ansprüche 1 bis 4,
wobei in dem Datenspeicher Informationen über eine zukünftige Reise gespeichert sind, wobei die Informationen einen Reisebeginn und optional eine Reisedauer umfassen
wobei die Verarbeitungseinheit konfiguriert ist, den Reisebeginn und optional die Reisedauer aus dem Datenspeicher auszulesen
wobei die Verarbeitungseinheit konfiguriert ist, zu einem definierten Zeitpunkt vor dem Reisebeginn die Ausgabeeinheit zu veranlassen, eine Mitteilung auszugeben, wobei die Mitteilung eine Erinnerung an die während der Reise einzunehmenden Arzneimittelportionen umfasst.

6. Vorrichtung gemäß Anspruch 5,
wobei die Verarbeitungseinheit konfiguriert ist, die Zahl der während der Reisedauer einzunehmenden Arzneimittelportionen zu ermitteln und die Ausgabeeinheit zu veranlassen, eine Mitteilung auszugeben, wobei die Mitteilung die Zahl der während der Reisedauer einzunehmenden Arzneimittelportionen umfasst.

7. Vorrichtung gemäß einem der Ansprüche 1 bis 6, ferner umfassend eine Empfangseinheit und eine Sendeeinheit,
wobei die Verarbeitungseinheit konfiguriert ist, mittels der Empfangseinheit Informationen über freie Termine für eine Untersuchung zu empfangen,
wobei die Verarbeitungseinheit konfiguriert ist, die Ausgabeeinheit zu veranlassen, die freien Termine anzuzeigen,
wobei die Verarbeitungseinheit konfiguriert ist, die Eingabeeinheit zu veranlassen, einen Terminwunsch durch den Nutzer entgegenzunehmen
wobei die Verarbeitungseinheit konfiguriert ist, den Terminwunsch mittels der Sendeeinheit zu versenden.

8. Vorrichtung gemäß Anspruch 7
wobei die Verarbeitungseinheit konfiguriert ist, Gesundheitsdaten über die Empfangseinheit zu empfangen
wobei die Verarbeitungseinheit konfiguriert ist, die Ausgabeeinheit zu veranlassen, Gesundheitsdaten anzuzeigen.

9. Vorrichtung gemäß einem der Ansprüche 1 bis 8,
wobei in dem Datenspeicher die Zeitpunkte und/oder Zeitspannen der Vergangenheit, zu denen der Nutzer eine oder mehrere Arzneimittelportionen einnehmen sollte, gespeichert sind,
wobei in dem Datenspeicher die Zeitpunkte der Vergangenheit, zu denen der Nutzer eine oder mehrere Arzneimittelportionen eingenommen hat, gespeichert sind,
wobei die Verarbeitungseinheit konfiguriert ist, das Verhältnis von eingenommenen zu einzunehmenden Arzneimittelportionen zu berechnen,
wobei die Verarbeitungseinheit konfiguriert ist, die Ausgabeeinheit zu veranlassen, das Verhältnis anzuzeigen.

10. Verfahren umfassend die Schritte:
- Ermitteln von Zeitpunkten und/oder Zeitspannen, zu denen ein Nutzer eine oder mehrere Arzneimittelportion im Rahmen einer klinischen Studie einnehmen soll
- Ausgeben einer Mitteilung über eine erforderliche Einnahme der oder der mehreren Arzneimittelportionen bei Eintreten eines definierten Ereignisses
- Anzeigen einer Abbildung der einen oder der mehreren Arzneimittelportionen auf einem Bildschirm.

11. Verfahren gemäß Anspruch 10, ferner umfassend die Schritte:
- Ausgeben einer Aufforderung zur Präsentation einer definierten Arzneimittelportion gegenüber einer Bildaufnahmeeinheit
- Aufnehmen einer Bildaufnahme von der präsentierten Arzneimittelportion
- Erkennen der Arzneimittelportion in der Bildaufnahme
- Vergleichen der erkannten Arzneimittelportion mit der definierten Arzneimittelportion
- Ausgeben einer Information über das Ergebnis des Vergleichens.

12. Verfahren gemäß Anspruch 10, ferner umfassend die Schritte:
- Ausgeben einer Aufforderung an den Nutzer, eine definierte Arzneimittelportion zu präsentieren,
- Erzeugen von Bildaufnahmen der präsentierten Arzneimittelportion und Anzeigen der Bildaufnahmen auf einem Bildschirm in Form von Live-Bilder
- Analysieren der Bildaufnahmen auf Anwesenheit einer Arzneimittelportion
- Anzeigen in den Live-Bildern, ob die Anwesenheit einer Arzneimittelportion erkannt wird oder nicht erkannt wird
- Identifizieren einer als anwesend erkannten Arzneimittelportion
- Vergleichen der identifizierten Arzneimittelportion mit der definierten Arzneimittelportion
- Ausgeben einer Mitteilung über das Ergebnis des Vergleichens.

13. Verfahren gemäß einem der Ansprüche 10 bis 12, ferner umfassend die Schritte:
- für den Fall, dass die identifizierte Arzneimittelportion mit der definierten Arzneimittelportion übereinstimmt:
- Ausgeben einer Mitteilung, wobei die Mitteilung eine Aufforderung zur Einnahme der Arzneimittelportion umfasst,
- Erfassen der aktuellen Zeit,
- Speichern einer Information über die Einnahme der Arzneimittelportion zu dem aktuellen Zeitpunkt in einem Datenspeicher.

14. Verfahren gemäß einem der Ansprüche 10 bis 13, ferner umfassend die Schritte:
- Erfassen von Zeitpunkten und/oder Zeitspannen der Vergangenheit, zu denen der Nutzer eine oder mehrere Arzneimittelportionen einnehmen sollte,
- Erfassen von Zeitpunkten der Vergangenheit, zu denen der Nutzer eine oder mehrere Arzneimittelportionen eingenommen hat,
- Berechnen des Verhältnisses von eingenommenen zu einzunehmenden Arzneimittelportionen,
- Anzeigen des Verhältnisses auf einem Bildschirm.

15. Computerprogrammprodukt umfassend einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:
- Ermitteln von Zeitpunkten und/oder Zeitspannen, zu denen ein Nutzer eine oder mehrere Arzneimittelportion im Rahmen einer klinischen Studie einnehmen soll
- Ausgeben einer Mitteilung über eine erforderliche Einnahme der oder der mehreren Arzneimittelportionen bei Eintreten eines definierten Ereignisses
- Anzeigen einer Abbildung der einen oder der mehreren Arzneimittelportionen auf einem Bildschirm.
